(19) Europäisches Patentamt
European Patent Office
Office européen des brevets

(11) **EP 2 138 174 A1**

(12) **EUROPEAN PATENT APPLICATION**
published in accordance with Art. 153(4) EPC

(43) Date of publication:
**30.12.2009 Bulletin 2009/53**

(51) Int Cl.:
*A61K 31/135* (2006.01)     *A61K 31/137* (2006.01)
*A61K 31/407* (2006.01)     *A61K 9/28* (2006.01)
*A61P 29/00* (2006.01)     *A61P 25/04* (2006.01)

(21) Application number: **08712559.7**

(22) Date of filing: **29.01.2008**

(86) International application number:
**PCT/MX2008/000011**

(87) International publication number:
**WO 2008/120966 (09.10.2008 Gazette 2008/41)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **02.04.2007 MX MX07003948**

(71) Applicant: **World Trade Import-Export, WTIE A.G. 6302 Zug (CH)**

(72) Inventors:
• **GARCÍA ARMENTA, María Elena**
**C.P. 45110 Zapopan, Jalisco (MX)**

• **SANTOS MURILLO, Josefina**
**C.P. 45110 Zapopan, Jalisco (MX)**
• **ALVAREZ OCHOA, Víctor Guillermo**
**C.P. 45110 Zapopan, Jalisco (MX)**

(74) Representative: **Carvajal y Urquijo, Isabel et al Clarke, Modet & Co.**
**Goya 11**
**28001 Madrid (ES)**

(54) **PHARMACEUTICAL COMPOSITION IN THE FORM OF A SUBLINGUAL TABLET CONSISTING OF A NON-STEROIDAL ANTI-INFLAMMATORY AGENT AND AN OPIATE ANALGESIC FOR PAIN MANAGEMENT**

(57) The invention relates to a pharmaceutical composition consisting of a combination of two active ingredients, one of said active ingredients being a non-steroidal anti-inflammatory agent, known as ketorolac, and the other being an opiate analgesic known as tramadol. Said agents are formulated in the form of an orally administered sublingual tablet used to relieve and/or treat moderately or severely intense pain. The combination of said pharmaceuticals generates a stronger analgesic effect, greater bioavailability and a faster speed of action using smaller dosages, with less collateral effects.

EP 2 138 174 A1

**Description**

**FIELD OF INVENTION**

The present invention has application in pharmaceutical industry and refers to a development of a pharmaceutical composition comprised by the association or synergic combination of an non-steroidal anti-inflammatory agent known as: Ketorolac trometamine and an opiate analgesic agent known as: Tramadol Hydrochloride, which are formulated in a single dose unit in the form of a sublingual tablet and being prescribed for patients having moderate to severe intensity pain.

The combination of said drug substances when jointly administered in a single dosage unit produces an analgesic synergy which provides as a result a larger analgesic effect unlike when these drug substances are independently administered, providing benefits such as: lower dosages, prevention of side effects which may be present when higher doses of drug substances are administered independently through other routes of administration, thus preventing a first step metabolism.

**BACKGROUND OF THE INVENTION**

Pain is one of the main and major causes of consultation in patients of any age, thus it is important to know the behavior and variations showed by this symptom. Commonly, patients expect until a severe or intolerable pain manifestation to attend a medical consultation. Pain may be a symptom or a consequence of a lesion, disease or surgery. There are many definitions intending to conceptualize pain meaning, one of these definitions recites: it is a sensorial perception of an unpleasant sensation associated with an emotional experience produced by current or potential tissular damage.

Pain perception and experience manifested by a patient are affected by many factors such as: its causes, patient cultural background, as well as prior experiences and emotions being these factors those which motivate that threshold and tolerance are variable in several persons and even in the same individual under different circumstances.

Above disclosed definition fully enlarges a belief that pain was simply an unpleasant nerve impulse and is replaced by the concept of experience, that is, an integration of all prior and current conditions before a nociceptive stimulus (nociception: noxa or damage reception). At pain moment itself, patient integrates prior experiences before similar stimuli, they are consciously processed and clarified with affective tone (always variable, depending on the circumstance where nociceptive episode is produced).

Perception is clearly objectionable and whether of sensorial (pure nociception) or emotional (going from a single affective component in perception until the own generation of painful perception by purely psychogenic mechanisms) nature, it is unpleasant and it always tend to be described in sensorial, tissular destruction, painful intensity, affection and/or assessment terms.

Pain is classified as: Acute Pain, which is of recent appearance with a generally but not always demonstrable cause, and may persist from minutes to days. A pain during more than 72 hours is called subacute. Chronic pain extends more than three months but this limit is quite arbitrary since it may be suffered by several months and even years, although its definition and causes may be variable.

Pain may have as a reference a distant origin site and it is common in internal organ or entrails affections such as kidneys, colon, uterus, and rectum and it may be referred to as lumbosacral region. Anatomic basis of referred pain are the somatic and visceral tissues which are enervated by afferent fibers from the same segment than spine root. Primary nociceptive ways are linked with other spinal segments and thus its perception may be located in a distant place from the site of actual disease. Nociceptive stimulus may produce hyperexcitability in nerve cells from spine cord which may refer pain to related tissues. Pain may be irradiated from its origin site, producing a large painful zone.

Pain perception starts with a stimulus from primary receptors which are found in skin or deep tissues. The two nociceptor types, A-delta and C fibers may respond to thermal stimuli, chemical signals or mechanical deformation. The number and type of pain receptors vary in several tissues; for instance, ligaments and periosteum are richly enervated by them and thus injuries in said areas are quite painful; on the other hand, the normal joint cartilage does not have pain receptors having the possibility to injure it severely without any pain manifestation. Visceral pleura does not have pain receptors either, which explains that abdomen-disseminated diseases may be little painful. When tissue is injured, several chemical substances such as histamines, prostaglandins and bradykinins are released which not only stimulate the pain receptor but make it also sensitive to future stimuli.

Once that any damage or wound occurs, the affected area becomes particularly sensitive and irritable and subsequently resulting ischemia in damaged tissues causes pain by a release of these chemical substances which when affecting nerve cells by a damage on blood vessels, is considered a cause of peripheral neuropathies such as in diabetic patients. Pain primary receptor communicates with spine cord through each nerve root and makes a synapse with other neurons before the painful stimulus moves upwards to higher centers such as thalamus and/or brain.

Interpretation of painful stimulus may be modified in spinal cord. It is the performance base of some treatments such as transcutaneous electric stimulation (TENS) and other sensitive counter-stimulation techniques. Centers such as thalamus, brain and spinal cord may also present modifications in pain appearance through descending inhibitory ways. Brain and spinal cord produce endogenous opiates such as encephalins and endorphins which reduce pain perception. Nerve tissue damage, both peripheral and central or both, may cause neurogenic pain. Experimental studies report that in the absence of a disease, there is no relative age to differentiate pain perception and tolerance; however, this must be cautiously understood since a change in pain perception in different people along their life span has not been well studied and these studies do not include very old patients (85 years or older), which is the age range where many diseases and more constant pain manifestations are expected.

Chronic pain has narrow psychological, emotional and sociological components, since it has demonstrated that there is a strong relationship between pain, depression and anxiety in groups of all ages. This relationship is complex, interactive and less understood. Long-term effects, in physical, psychological and social welfare for any individual, make that a chronically painful person is more prone to depression especially if he/she is older. Similarly, a depressed sick person may delay more in recovery and this may alter his/her experience in the face of pain. High levels of unconditioning or physical limitations are other factors associated with pain and depression.

Painful diseases are of greater interest in older adults than in younger ones, since older adults are more exposed to conditions which cause a pain manifestation in both types: acute and chronic. Surgical procedures are more frequently performed in older adults since they are more predisposed to have long evolution diseases associated with significant pain such as: osteoarthritis, osteoporosis with vertebral fractures, diabetes with peripheral neuropathy, cancer, peripheral vascular disease, post-herpetic neuropathy, etc.

There are currently a large variety of useful pharmaceutical products for pain treatment; however, drug substances are independently administered on said drugs causing that disorder relief is slower and less effective.

Ketorolac is a non-steroidal anti-inflammatory agent presenting analgesic, anti-inflammatory and antipyretic properties; which has been used in patients having pain and which may be orally, intramuscularly or intravenously administered. This drug substance inhibits prostaglandin synthesis and it is a potential analgesic with peripheral activity. It has a moderate anti-inflammatory effect.

It has been demonstrated that ketorolac shows a higher analgesic efficiency compared with the effect manifested by opiates (according to clinical studies reported by Yee, et al in 1986; O'Hara, et al in 1987; Forbes, et al in 1990). Analgesic activity of 30 mg of ketorolac is equivalent to 9 mg of morphine but without causing side effects such as: drowsiness, nausea, vomit and without respiratory effects. Analgesic effect of 10 to 20 mg of ketorolac orally administered is equivalent to 650 mg of acetylsalicylic acid or 600 mg of acetaminophen with 60 mg of codeine. Unlike opiates, ketorolac does not decrease the inhaled anesthetic CAM. Upon administering clinical doses of ketorolac, manifestations of cardiovascular or respiratory effects are not shown. Ketorolac inhibits platelet aggregation, an inhibition which disappears from 20 to 40 hours after withdrawing medicament without altering the platelet count, prothrombin time or thromboplastin partial time.

It has been demonstrated that ketorolac is effective in short-term treatment for moderate to severe intensity pain (Bloomfield et al 1986), caused by several etiologies, mainly in clinical profiles where a powerful and sustained analgesic activity is required, without showing side effects manifested by opiate analgesic administration. It is prescribed for treatment of post-surgical, traumatic, orthopedic, rheumatologic, gynecologic, urologic, neurologic, oncologic, teeth pain and in renal and biliary colic.

Ketorolac intervenes peripherally in biochemical mechanisms which cause a painful sensation, blocking the pain localization and propagation until marrow and brain nerve centers. Pain intensity is rapidly released or fully removed.

Ketorolac acts inhibiting to cyclooxigenase enzyme with prostaglandin peripheral synthesis inhibition, which intervene in the inflammatory and painful process, reducing pain and inflammation. Analgesic effect is basically peripheral without altering pain threshold. Its anti-thermal action is exerted by prostaglandin inhibition which mediates the effect of pyrogen endogenous effects at hypothalamus level. Moreover, it induces a decrease in gastric mucosa cytoprotection, renal blood flow decrease, as well as platelet aggregation inhibition. It presents a discrete anticholinergic and $\alpha$-blocker activity. It is rapidly and fully absorbed, reaching maximum plasma concentrations between 30 and 60 minutes after its administration. After oral and intramuscular administration, its analgesic effect starts at 10 and 30 minutes, respectively, with a period of 6 to 8 hour in both cases. Ketorolac crosses the placenta and is secreted by mother's milk. It has a plasma half-life of 3.5 to 8.6 hours. It is metabolized by liver and about 91% of dose is removed by urine without any change, the remaining being excreted in fecal feces.

Tramadol is an opiate type (narcotic) central action analgesic. It is a $\mu$-opiate receptor weak agonist having noradrenergic properties, inhibiting noradrenaline relocation; and serotoninergic properties, inhibiting serotonin release in nerve terminations, which contribute to analgesic activity.

Tramadol has been used in Europe since 70's and in the United States since late 80's to relief many types of painful conditions. Tramadol is used for relief of moderate to severe intensity pain and it acts over spine cord and brain specific nerve cells which decrease a natural function which is increased upon administering this drug.

Tramadol may be orally, intramuscularly or intravenously administered. Maximum plasma concentrations are reached between 1.5 and 2 hours after oral administration and possess through this way a 79% bioavailability. It is metabolized in liver producing active metabolites such as M1. Analgesic effect half-life of Tramadol is about 6 hours after each dose delivery and analgesic effect start is manifested when elapsing 10 to 20 min after administration, being mostly removed in urine.

Tramadol is used for treatment of pain manifestations caused by: surgeries (post-operation), joint traumatisms (post-trauma), chronic conditions such as cancer, neuropathy pain and others; and it functions by decreasing body sensitivity to said pain manifestations.

There are currently a large variety of medicaments for pain treatment, those which are administered orally in solid form. The pharmaceutical composition subject of present invention is formulated in a single dose unit in a form of a sublingual tablet to be orally administered.

Sublingual tablets are designed to rapidly dissolve in oral fluids, thus obtaining a higher speed and fastness in drug substance absorption contained in a pharmaceutical composition. Disintegration mechanism of these medicaments is carried out by dissolving them under the tongue or between the gums and cheek to perform a fast absorption of drug substances and they reach blood circulation in a time period as short as possible. If these pharmaceutical compositions in sublingual tablet form are swallowed, their therapeutic effect results ineffective or less effective regarding the effect obtained when correctly administered.

## SUMMARY OF INVENTION

The object of present invention is centered in a development of a pharmaceutical composition comprising a synergic combination of a non-steroidal anti-inflammatory agent with analgesic activity known as: Ketorolac and an opiate analgesic agent such as Tramadol, those which are formulated in a single dose unit to be orally administered in the form of a sublingual tablet, which provides benefits such as a lower dosage of drug substances, higher fastness in drug substance absorption and preventing the manifestation of side effects which are commonly present when administering drug substances independently and at larger doses.

## DETAILED DESCRIPTION OF THE INVENTION

Historically, the fact of associating or combining drug substances which have similar or different therapeutic activity applied to pain management has been a matter of controversy.

The technical foundation which justifies this clinical practice is easy to state. Anti-inflammatories are drugs which base its analgesic power in their capacity to inhibit prostaglandins thus reducing inflammation, while central analgesics upon interacting with its specific receptors produce an alteration in neuron membrane permeability in such a way that selectively inhibit the nociceptive impulse, thus being pure analgesics.

In recent years, a very important trend has been manifested for pharmaceutical composition research and development comprising a combination of drug substances with specific analgesic activity, formulated in a single dose unit having the purpose of achieving an analgesic synergy where lower drug substance doses are used, and providing as a result a reduction in manifested side effects unlike when said drug substances are independently administered and in larger doses.

On the other hand, a significant emphasis has been generated in the development of new pharmaceutical forms which increase the absorption speed of formulated drug substances and which further increase its bioavailability and therapeutic efficacy with lower risks manifested in side effects.

Development of present invention is based in a synergic combination of a non-steroidal anti-inflammatory agent known as: Ketorolac and an opiate analgesic agent such as: Tramadol, those which are formulated in a single dose unit to be orally administered in a sublingual tablet form, indicated for pain treatment with moderate to severe intensity.

Ketorolac is manifested as a non-steroidal anti-inflammatory (AINE) with common features, that is, a high anti-inflammatory power and therefore a marked analgesic action. Tramadol on the other hand is not shown as a typical central analgesic since its central action is associated with the capacity to inhibit noradrenaline and 5-hydroxytriptamine reuptake, thus providing a novel profile to the pharmaceutical composition object of present invention as to its efficient analgesic power, thus keeping between up to now unknown safety margins in central analgesics. From the association of these two drug substances with different action mechanisms, which were formulated as a sublingual tablet form, quite satisfactory results which are below described were obtained.

We started our research performing preclinical trials which firstly demonstrated a combination synergy.

## EXAMPLES

### Example 1

In order to determine the possibility of an antinociceptive synergic interaction, ketorolac trometamine, p.o., a non-steroidal anti-inflammatory (AINE) and tramadol hydrochloride, p.o., an atypical analgesic opiate, antinociceptive effects were determined, administered both separately and in combination. A rat arthritic pain model was determined for said purpose. Data were interpreted by using a Surface Synergic Interaction Analysis (ISS) and an Isobolographic Analysis to determine the interaction nature. ISS is calculated from the total antinociceptive effect produced by a combination after a substraction of an antinociceptive effect produced separately by the drug. Female rats received ketorolac orally independently, Tramadol orally independently, or 24 combinations of ketorolac plus tramadol.

### Material and methods.

For this trial, Wistar [CRI (WI) BR] female rats weighing 180-200 g. were weighed. Feed was maintained 12 hours before the water ad libitum access experiments. All the experimental procedures followed the recommendations of the Committee for Research and Ethical Issues of the International Association for the Study of Pain (Covino et al., 1980) and Guidelines on Ethical Standards for Investigations of Experimental Pain in Animals (Zimmermann, 1983) and they were carried out according to a protocol approved by the local Animal Ethics Committee. Animal number in experiment was kept at a maximum and animals were maintained in a weathered room and light control with a 12-hour light/darkness cycle.

### Medications

Uric acid was suspended in mineral oil; Ketorolac trometamine, tramadol hydrochloride and indomethacin were dissolved in carboxymethyl cellulose and orally administered.

### Antinociceptive activity measurement.

Antinociceptive activity was determined by using a PIFIR model which has been described in detail (López-Muñoz et al., 1993). Animals were anesthetized with chamber ether (Pyrex-glass dryer saturated with ether vapor). Nociception was induced through an intraarticular injection (ia) of 0.5 mL uric acid suspended in mineral oil in the right-hind leg knee joint. Suspension was prepared by mixing 3.0 g of uric acid with 10 mL of mineral oil in a glass mortar with pestle (Pyrex). Intraarticular injection was carried out through the patellar ligament using a 1 mL glass syringe (Beckton Dickinson LTA, Brazil) with a 5 mm 24-needle. Immediately after, an electrode was implanted in the plantar surface of each hind claw between the plantar pads. Rats were allowed to recover from anesthesia and then a stainless steel cylinder was attached which was rotated at 4 rpm, forcing to the rats to walk for 2 minute periods every 30 minutes during 6.5 hours. It was not necessary a training period since rats learned it from the first minute. Contact time between each implanted electrode in rat limb and cylinder was registered. After the uric acid injection, rats progressively developed damaged limb dysfunction. Contact time of damaged limb reached a zero value after 2.5 hours of uric acid injection; in this time Ketorolac and Tramadol alone or in combination were administered. This time was considered as zero time for antinoceptive effect measures; these effects are measured every 30 minutes during the following 4 hours. This allowed determining the course of the nociceptive effects in the same animal. Antinociception was considered as a recovery of contact time. Data are expressed as a performance percentage index (IF%; that is, contact time of injected leg divided by contact time of left leg, control, multiplied per 100). For the purpose of this trial, it was unavoidable to induce nociception in experimentation animals. However, care was taken in preventing an unnecessary suffering. All the experiments were performed between 7:00 A.M. and 2:00 P.M.

### Study Design

Nociceptive effects produced by ketorolac and tramadol whether administered independently or in combination were studied. Firstly, each Ketorolac dose (0.18, 0.32, 0.56, 1.0, 1.78, 3.16 or 5.62 mg/kg) or tramadol dose (3.16, 5.62, 10.0, 17.78, 31.62, 56.23 or 100.0 mg/kg) was delivered to six animals to obtain the corresponding dose response curves and ketorolac doses (0.10, 0.18, 0.32, 0.56, 1.0, or 1.78 mg/kg) and tramadol (3.16, 5.62, 10.0, or 17.78 mg/kg) were then combined to analyze possible synergic interactions (24 total combinations). At the end of the study the rats were sacrificed.

Gastrointestinal effect measurements

Female rats from Wistar type (150-180 g body weight) were subject to fasting 24 hours before experimentation. Indomethacin was supplied (20 mg/kg) to cause gastric ulcer at 100% (Lee et al., 1971 Déciga-Campos et al., 2003); ketorolac (1.0 mg/kg), tramadol (17.8 mg/kg), vehicle (carboxymethylcellolose at 0.5%) and a combination of ketorolac plus tramadol (1.0 and 17.8 mg/kg respectively) was orally delivered simultaneously and to the five groups (six rats each). 2.5 hours later, all the groups received a second administration of the same doses. Stomachs were examined at 5 hours after first treatment in the following way: animals were sacrificed and stomach was separated which was opened along its lower curvature, gently rinsing with formol (2%) and examined. Severity of induced gastric lesions with medicament was calculated as a ratio of number of lesions (stomach ulcer or erosion) caused by provided treatment and the number of lesions caused by indomethacin (100%). This was determined to reflect the side effects induced by medicament. The sum of the area of all the ulcers in the body of each animal was calculated and served as an ulcerative index. Gastric lesion percentage was calculated in the following way:

$$\texttt{\% of gastric lesion = (IUM/IUI) x 100}$$

wherein IUM is the Medicament Ulcerative Index on test ($mm^2$) and IUI is the Ulcerative Index for assayed Indomethacin ($mm^2$).

Data presentation and statistic assessment

Study data, tables and figures are expressed as IF%. IF% vs. time curves were prepared for each treatment and the corresponding time course was obtained. Antinociception was estimated as a recovery of IF%. Accumulated antinociceptive effect during all the observation period (4 hours) was determined as the area under the curve (ABC) for time course to obtain the dose-response curve and for analyzing the total antinociceptive effect obtained for the analgesic agent whether alone and in combination.

Synergism between ketorolac and tramadol was calculated with the synergic interaction surface analysis (SIS) and with the isobolographic method (Tallarida et al., 1989). ABC was calculated for each drug combination and for each of the components. On the basis of addition of individual pharmacological effects (Seegers et al., 1981) an ABC equivalent to the sum was expected. If the sum of the corresponding individual ABC was higher than the theoretical sum, the result was determined as enhancement; when similar to the theoretical sum it was determined to show additive antinociceptive effects. ABC was obtained through the trapezoidal rule (Rowland and Trozer, 1989). All the values for each treatment were average $\pm$ S.E.M., for six animals. ABC values for drug combinations were compared with the values which were expected by using the Student test. Obtained ABC values from antinociceptive effects produced by ketorolac or tramadol (independently assayed) were compared with the ABC values obtained from the corresponding combination through a variance analysis (ANOVA) and Dunnett test. Gastrointestinal side effects caused by ketorolac or tramadol (whether separately or when combined assayed) were compared with the gastrointestinal effects obtained from indomethacin through Dunnett test. A P<0.05 was determined as statistically significant.

Doses which caused 50% of maximum possible effect ($DE_{50}$) for each drug were calculated by performing a linear regression analysis of the linear portion from dose-response curves. An isobologram was prepared by using $DE_{50}$ when drugs were independently or combined delivered. In order to perform the isobolographic analyses, ketorolac and tramadol were administered in combination as fixed rates of $DE_{50}$ efficient dose for each drug (ketorolac:tramadol=1: 1). $DE_{50}$ values ($\pm$ S.E.M.) for ketorolac and tramadol were detailed over x- and y- axis, respectively and theoretical additive point was calculated according to Tallarida et al., (1989). $DE_{50}$ value was calculated from the total combination dose from the dose-response curve for the combined drugs. Statistical significance was validated between the theoretical additive point and $DE_{50}$ experimental value using Student test. A $DE_{50}$ experimental figure significantly lower than $DE_{50}$ theoretical addition (P<0.05) was considered to indicate a synergic interaction between ketorolac and tramadol.

Results

Uric acid and vehicle effect

Uric acid induced a complete dysfunction in the hind right leg corresponding to an IF% value of zero in 2.5 hours. This dysfunction was maintained along the 4-hour experimental period. Rats which received vehicle (carboxymethylcellulose at 0.5%) did not show any significant IF% recovery during the observation term. Used doses for ketorolac (0.18 -5.62 mg/kg) and tramadol (3.16-56.23 mg/kg) did not affect the walking capacity of rats treated with vehicle (data are not shown). Tramadol at 100 mg/kg, produced side effects in 50% of animals, sedation, elation, dizziness and effects on walking capacity in rats.

<u>Antinociceptive effects of individually assayed drugs</u>

Figure 1 shows the dose-response curves for ketorolac and tramadol. Both drugs increased ABC in a dose-dependent form but they showed a different efficacy (i.e., ketorolac caused a maximum efficiency). Therefore, ketorolac (2.6 mg/kg) showed a great antinociceptive efficiency of 295.8 $\pm$ 14.9 au compared with tramadol (56.23 mg/kg) which showed an antinociceptive activity of 207.7 $\pm$ 24.7 au. $DE_{50}$ values for drugs indicate that there were significant differences in antinociceptive potencies: ketorolac ($DE_{50}$=0.86 $\pm$ 0.10 mg/kg) was more potent than tramadol ($DE_{50}$= 44.29 $\pm$ 0.06 mg/kg). There were no adverse effects with used doses. Antinociceptive effects shown by tramadol at 100 mg/kg (236.7 $\pm$ 11.0 au) are not included in Figure 1 since it caused side effects in rats.

<u>Antinociceptive effects of medicament combinations</u>

Figures 2 to 4 disclose the antinociceptive effect of 24 combinations showed over three-dimensional charts. These were prepared using an average of six animals per each administered dose whether independently or in combination. The maximum antinociceptive effect which may be obtained from several ketorolac + tramadol combinations (1.78 + 17.78 mg/kg), respectively; see fig. 2) was 372.7 $\pm$ 15.6 au.

Statistical analysis from data in figure 2 indicate an interaction between ketorolac and tramadol (P<0.05) while there were no side effects in the assayed combination.

Figure 3 was produced in order to distinguish between additive effects and enhancement effects. This chart was calculated from the total antinociceptive effect caused by combinations after the antinociceptive effect deduction caused by each component administered independently. Results higher than level "0" were considered as showing enhancement, while those with level "0" were considered addition. Although this type of mapping allows the observation of antinociceptive antagonist effects, these were not obtained in present study. Likewise, 14 combinations of ketorolac and tramadol caused antinociceptive effects and 10 caused enhancement with 95% confidence limits (P<0.05) (Fig. 3); these combinations were: 17.78 mg/kg tramadol whether with, 0.18, 0.32 or 0.56 mg/kg ketorolac; 10.0 mg/kg tramadol with whether 0.18, 0.32, 0.56 or 1.78 mg/kg ketorolac; 5.62 mg/kg tramadol with whether 0.32 or 0.56 mg/kg ketorolac and 3.16 mg/kg tramadol with 0.32 mg/kg ketorolac. In order to obtain the synergic interaction surface for ketorolac and tramadol combinations, all interaction points from Fig. 3 were unified in a plane. The result is a synergic interaction surface from these analgesic medicaments shown in Figure 4. By using this chart, it is easy to note the medicament interactions of Ketorolac + Tramadol (i.e., addition or enhancement). For instance, 10 Ketorolac + Tramadol combinations showed different degrees of enhancement of antinociceptive effects, but 2 combinations of Ketorolac + Tramadol showed high enhancement effects (0.32 $\pm$ 10 and 0.56 + 10 mg/kg respectively) (see Table 1). Tramadol at a dose of 10.0 mg/kg provided an ABC of 22.5 $\pm$ 9.3 au and Ketorolac at a dose of 0.32 mg/kg showed an ABC of 65.0 $\pm$ 9.6 au; however, Ketorolac + Tramadol combination (10 + 0.32 mg/kg) allowed an AUC of 233.5 $\pm$ 25.8 au, which is higher than expected ABC resulting from the sum of individual values (i.e., 87. 5 au) (P<0.001). $E_{max}$ analysis of corresponding time course curves showed an increase in values obtained from combinations (86.6 $\pm$ 6.3 %) which was higher than the corresponding values (Ketorolac 33.7 $\pm$ 8.7% and Tramadol 17.1 $\pm$ 9.1%) obtained from the arithmetic sum (50.8%). Other enhancement examples for Ketorolac + Tramadol are shown in table 1.

Antinociceptive effects obtained from combinations which caused the maximum antinociceptive effect (1.78 mg/kg Ketorolac + 17.78 mg/kg Tramadol) and the combination which produced the highest enhancement (0.56 mg/kg Ketorolac + 10.0 mg/kg Tramadol) is shown in Fig. 5. As it can be seen from Fig. 5A, antinociception caused by Ketorolac + Tramadol (1.78 + 17.78 mg/kg) represented the maximum antinociceptive effect (representing a total recovery) obtained with 372.7 $\pm$ 15.6 au, while Ketorolac independently administered (1.78 mg/kg) showed an ABC of 279.0 $\pm$ 15.5 au, and Tramadol independently administered (17.78 mg/kg) produced only 58.2 $\pm$ 23.7 au. This result was important when considering that maximum used ketorolac dose (3.16 mg/kg) caused a lower antinociceptive effect: 295.8 $\pm$ 14.9 au. The combination described in Fig. 5B (0.56 mg/kg of ketorolac + 10.0 mg/kg Tramadol) represents only a combination which produced the maximum enhancement of antinociceptive effect (169.4% plus ABC or full antinociceptive effect compared with the sum of individual ABC); moreover, both time course and obtained ABC with this combination were higher (P<0.001) than the respective values obtained with the sum of individual agents (121.2 au). Antinociception caused by Ketorolac/Tramadol (0.56 + 10.0 mg/kg) was 290.6 $\pm$ 27.7 au, while Ketorolac independently administered (0.56 mg/kg) showed an ABC of 98.7 $\pm$ 15.5 au and Tramadol independently administered (10.0 mg/kg) produced only 22.5 $\pm$ 9.3 au. A significant antinociceptive effect was obtained with the combination along all the observation period (4 hours). Another approach for research of synergic interaction between two selected analgesic drugs is the isobolographic method, Tallarida et al., 1989. An isobologram is shown in Fig. 6 wherein the antinociceptive interaction between Ketorolac and Tramadol may be noticed in a model of functional impairment induced by pain in rats. Horizontal and vertical bars indicate the S.E.M. Oblique line between "x-" and "y-" axes is the additive theoretical line. Mean point from this line is the theoretical additive point calculated from separated DE values. Experimental point is located far below the additive line, indicating an additive synergism (P<0.05).

Measurement of gastrointestinal side effects

Tramadol administration did not cause ulcers or erosions. Its side effects were similar to those from vesicle. However, Ketorolac generated a lower ulcer area (13.7 $\pm$ 2.7 mm$^2$) and lower erosion number (15.0 $\pm$ 4.9) than indomethacin (P<0.05), which was considered as the most nocive component in terms of number and severity of lesions caused in stomach (i.e, ulcers and erosions) (100%). Ketorolac + Tramadol combination generated less ulcers (16.9 $\pm$ 6.9 mm$^2$) and a lower number of erosions (5.0 $\pm$ 1.4) than indomethacin (P<0.05). It is worth to note that Ketorolac + Tramadol combination reduced erosion generation (P<0.05) and ulcerations were similar to those produced by Ketorolac independently administered (Table 2).

Table 2

| Gastrointestinal side effect comparison expressed as a percentage of gastric lesion produced by indomethacin (20 mg/kg), Ketorolac (1 mg/kg), Tramadol (17.8 mg/kg) and a combination of Ketorolac and Tramadol (1 and 1.78 mg/kg, respectively). | | | |
|---|---|---|---|
| Treatment (%) | No. of erosions | Ulcers[a] (mm$^2$) | Gastric lesion[b] |
| Vehicle | 0 | 0 | 0 |
| Indomethacin | 30.0 $\pm$ 3.9 | 51.6 $\pm$ 3.4 | 100 |
| Ketorolac | 15.0 $\pm$ 4.9[c] | 13.7 $\pm$ 2.7[c] | 26.6 |
| Tramadol | 0 | 0 | 0 |
| Ketorolac/ Tramadol | 5.0 $\pm$ 1.4[c] | 16.9 $\pm$ 6.9[c] | 32.8 |
| - [a]Sum of the area of all ulcers<br>- [b](Index of ulcers in drug tests / Index of ulcers in indomethacin tests) x 100, wherein the index from area in all stomach ulcers.<br>- [c]ANOVA (Dunnet test), P<0.05 regarding indomethacin. | | | |

## Example 2

Bioavailability of two oral and one sublingual formulations was compared, after a 40 mg + 100 mg Ketorolac trometamine /tramadol hydrochloride single dose, respectively, which was administered after fasting at least 10 hours in two independent sessions, with a range of one lavage week. Blood samples were taken up to 24 hours after medicament delivery. Plasma concentrations for Ketorolac trometamine and tramadol hydrochloride were determined using a method of high resolution liquid chromatography.
Plasma concentration profiles regarding time demonstrated a similar behavior, obtaining the pharmacokinetic parameters illustrated below.

**Pharmacokinetic parameters for ketorolac trometamine**

| | Cmax (ng/mL) | | ABC $0\rightarrow\infty$(hr*ng/ml) | | Tmax (hr) | |
|---|---|---|---|---|---|---|
| | Presentation | | Presentation | | Presentation | |
| | Cap | Tab | Cap | Tab | Cap | Tab |
| Mean | 8920.79 | 7180.27 | 35566.97 | 35566.97 | 0.79 | 0.88 |
| SD | 2719.20 | 2091.23 | 9928.89 | 9928.89 | 0.50 | 0.50 |
| CV% | 30.5 | 29.1 | 27.9 | 27.9 | 63.9 | 57.3 |
| | Geometric mean | | | | | |

(continued)

|  | Cmax (ng/mL) | | ABC 0→∞(hr*ng/ml) | | Tmax (hr) | |
|---|---|---|---|---|---|---|
|  | Presentation | | Presentation | | Presentation | |
|  | Cap | Tab | Cap | Tab | Cap | Tab |
|  | 8516.77 | 16867.32 | 34311.24 | 34311.24 | 0.69 | 0.77 |

**Pharmacokinetic parameters for Tramadol hydrochloride**

|  | Cmax (ng/mL) | | ABC 0→∞(hr*ng/ml) | | Tmax (hr) | |
|---|---|---|---|---|---|---|
|  | Presentation | | Presentation | | Presentation | |
|  | Cap | Tab | Cap | Tab | Cap | Tab |
| Mean | 412.635 | 562.855 | 3345.732 | 4145.427 | 1.462 | 1.106 |
| SD | 132.632 | 174.889 | 1964.353 | 1757.071 | 0.780 | 0.867 |
| CV% | 32.1 | 31.1 | 58.7 | 42.4 | 53.4 | 78.4 |
|  | Geometric mean | | | | | |
|  | 388.995 | 1534.449 | 2924.952 | 3829.342 | 1.233 | 0.889 |

In order to determine whether bioavailability is existent or not between both medicaments, a logarithmic transformation of data was carried out and they were analyzed by means of the statistical package WinNonlinTM.

Confidence ranges proposed by FDA for semilogarithmic data in determining bioequivalency of two products are from 80 to 125%. Classic and Westlake confidence ranges obtained for Cmax, ABC 0-t and ABC 0 → ∞ were:

**Statistical analysis for pharmacokinetic parameters of ketorolac trometamine**

| Dependent Power | Units | Difference | Ratio (%Ref) | C190 Lower | C190 Upper | WL90 Lower | WL90 Upper | Ahpval |
|---|---|---|---|---|---|---|---|---|
| Ln (Cmax) 0.9101 | ng/mL | 0.2153 | 72.78 | 109.61 | 140.32 | 63.61 | 136.39 | 0.4570 |
| LnAUCINF 0.9999 | ng/mL | -0.0457 | 95.53 | 89.96 | 101.45 | 91.20 | 108.80 | 0.0000 |

**Statistical analysis for pharmacokinetic parameters of Tramadol hydrochloride**

| Dependent Power | Units | Difference | Ratio (%Ref) | C190 Lower | C190 Upper | WL90 Lower | WL90 Upper | Ahpval |
|---|---|---|---|---|---|---|---|---|
| Ln (Cmax) 0.9101 | ng/mL | -0.3177 | 124.02 | 64.29 | 82.40 | 66.15 | 133.85 | 0.8975 |
| LnAUCINF 0.9999 | ng/mL | -0.2694 | 76.38 | 70.26 | 83.04 | 71.62 | 128.38 | 0.8235 |

Values from Imax quotient (Tab/Cap) is 124.02 which involves that tablet is faster absorbed in 24% and in order to show a bioequivalency between two pharmaceutical forms, calculated intervals (109.61 to 140.32) shall be comprised between 80 and 120%, which resulted not to be true for Imax, this involves that tablet presents a larger bioavailability as to absorption speed and possibly presents a faster therapeutic effect. However, it is not the same for ABC→∞ which represents the absorbed amount and both pharmaceutical forms may be considered as bioequivalent.

Conclusion

Formulations for ketorolac trometamine 10 mg and tramadol hydrochloride 25 mg combination in sublingual tablets show a larger bioavailability referred to absorption speed but they are bioequivalent in the absorbed amount compared to the capsule combination.

**Claims**

1. Pharmaceutical composition comprising a synergic combination of a non-steroidal anti-inflammatory agent and an opiate analgesic agent, **characterized in that** the non-steroidal anti-inflammatory agent is the drug substance: Ketorolac trometamine and the opiate analgesic agent is the drug substance: Tramadol hydrochloride, those which are formulated in a single dose unit in a sublingual tablet form, which has higher bioavailability, therapeutic action is more effective and shows less side effects.

2. Pharmaceutical composition in accordance with claim 1, **characterized in that** the drug substance: Ketorolac trometamine is present in the formulation in a concentration range from 0.0010 to 0.10000 g per dose unit.

3. Pharmaceutical composition in accordance with claim 1, **characterized in that** the drug substance: Tramadol hydrochloride is present in the formulation in a concentration range from 0.0010 to 0.20000 g per dose unit.

4. Pharmaceutical composition in accordance with claims 1 to 3, **characterized in** being formulated in a single dosage unit wherein a synergy is produced in such a way that provides as a result an incorporation of lower concentrations of each drug substances, those which are formulated to be orally administered in a sublingual tablet form.

5. The use of a pharmaceutical composition in accordance to claims 1 to 4, **characterized in that** being prescribed for moderate to severe intensity pain relief and/or treatment caused by: cephalea, teeth disturbances, sport injuries, surgeries as well as chronic diseases such as: cancer or joints, in addition to other conditions causing pain.

# INTERNATIONAL SEARCH REPORT

| International application No. |
|---|
| PCT/ MX 2008/000011 |

**A. CLASSIFICATION OF SUBJECT MATTER**

see extra sheet

According to International Patent Classification (IPC) or to both national classification and IPC

**B. FIELDS SEARCHED**

Minimum documentation searched (classification system followed by classification symbols)

Documentation searched other than minimum documentation to the extent that such documents are included in the fields searched

Electronic data base consulted during the international search (name of data base and, where practicable, search terms used)

INVENES, EPODOC, WPI

**C. DOCUMENTS CONSIDERED TO BE RELEVANT**

| Category* | Citation of document, with indication, where appropriate, of the relevant passages | Relevant to claim No. |
|---|---|---|
| Y | US 2004087644 A (GARCIA ARMENTA M.E. Y COL.) 06.05.2004, Page 2, paragraph [0014]. | 1-7 |
| Y | WO 2004108110 A1 (SILVESTRINI B.& BONANOMI M.) 16.12.2004, claims 1 and 7; page 4, lines 7-19. | 1-7 |
| A | EP 546676 A1 (MCNEILAB INC) 16.06.1993. Page 3, lines 27- page 4, lines 46; examples 1, 2 y 3; claims. | 1-7 |
| A | US 5914129 A (MAUSKOP ALEXANDER) 22.06.1999, claims | 1-7 |

☐ Further documents are listed in the continuation of Box C.   ☒ See patent family annex.

| | | |
|---|---|---|
| * Special categories of cited documents: | "T" | later document published after the international filing date or priority date and not in conflict with the application but cited to understand the principle or theory underlying the invention |
| "A" document defining the general state of the art which is not considered to be of particular relevance. | | |
| "E" earlier document but published on or after the international filing date | | |
| "L" document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" document referring to an oral disclosure use, exhibition, or other means | "Y" | document of particular relevance; the claimed invention cannot be considered to involve an inventive step when the document is combined with one or more other documents , such combination being obvious to a person skilled in the art |
| "P" document published prior to the international filing date but later than the priority date claimed | | |
| | "&" | document member of the same patent family |

| Date of the actual completion of the international search | Date of mailing of the international search report |
|---|---|
| 25 April 2008      (25.04.2008) | **(21/05/2008)** |
| Name and mailing address of the ISA/ O.E.P.M.<br><br>Paseo de la Castellana, 75 28071 Madrid, España.<br>Facsimile No.   34 91 3495304 | Authorized officer<br>       E. Albarrán Gómez<br><br>Telephone No. +34 91 349 30 38 |

Form PCT/ISA/210 (second sheet) (April 2007)

INTERNATIONAL SEARCH REPORT

International application No.

Box No. II    Observations where certain claims were found unsearchable (Continuation of item 2 of first sheet)

This international search report has not been established in respect of certain claims under Article 17(2)(a) for the following reasons:

1. ☒ Claims Nos.: 5
   because they relate to subject matter not required to be searched by this Authority, namely:

   Claim 5 relates to a method for treatment of the human or animal body by therapy. The
   search was carried out and was based on the possible effects of the composition.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Box No. III    Observations where unity of invention is lacking (Continuation of item 3 of first sheet)

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

Remark on Protest    ☐ The additional search fees were accompanied by the applicant's protest and, where applicable, the payment of a protest fee.
    ☐ The additional search fees were accompanied by the applicant's protest but the applicable protest fee was not paid within the time limit specified in the invitation.
    ☐ No protest accompanied the payment of additional search fees.

Form PCT/ISA/210 (continuation of first sheet (2)) (April 2005)

## INTERNATIONAL SEARCH REPORT

Information on patent family members

International application No.

PCT/ MX 2008/000011

| Patent document cited in the search report | Publication date | Patent family member(s) | Publication date |
|---|---|---|---|
| US 2004087644 A | 06.05.2004 | MX P | 16.07.2004 |
| WO 2004108110 A | 16.12.2004 | EP 1631253 A<br>US 2006141027 A | 08.03.2006<br>29.06.2006 |
| EP 0546676 AB | 16.06.1993 | CA 2081604 AC<br>NO 924169 A<br>AU 2732892 A<br>CN 1073095 A<br>EP 19920309930<br>HU 63557 A<br>MX 9206257 A<br>ZA 9208372 A<br>ZW 17892 A<br>JP 6157301 A<br>NZ 244916 A<br>US 5516803 A<br>IL 103594 A<br>EG 20041 A<br>PH 31378 A<br>SG 55086 A<br>KR 100236562 B<br>AT 285237 T<br>DK 546676 T<br>PT 546676 T<br>ES 2235152 T<br>DE 69233462 T | 01.05.1993<br>03.05.1993<br>06.05.1993<br>16.06.1993<br>29.10.1992<br>28.09.1993<br>01.11.1993<br>29.04.1994<br>01.06.1994<br>03.06.1994<br>27.04.1995<br>14.05.1996<br>18.03.1997<br>27.03.1997<br>29.10.1998<br>21.12.1998<br>02.03.2000<br>15.01.2005<br>07.03.2005<br>31.03.2005<br>01.07.2005<br>16.02.2006 |
| US 5914129 A | 22.06.1999 | WO 9803179 A<br>AU 3665897 A<br>EP 19970933490 | 29.01.1998<br>10.02.1998<br>21.07.1997 |

Form PCT/ISA/210 (patent family annex) (April 2007)

## INTERNATIONAL SEARCH REPORT

International application No.

PCT/ MX 2008/000011

CLASSIFICATION OF SUBJECT MATTER

*A61K 31/135* (2006.01)
*A61K 31/137* (2006.01)
*A61K 31/407* (2006.01)
*A61K 9/28* (2006.01)
*A61P 29 /00* (2006.01)
*A61P 25/04* (2006.01)

Form PCT/ISA/210 (extra sheeet) (April 2007)